Europäisches Patentamt

European Patent Office

Office européen des brevets

(19)

(11) Publication number: 0 350 913
A1

# EUROPEAN PATENT APPLICATION

(21) Application number: 89112839.9

(22) Date of filing: 13.07.89

(51) Int. Cl.⁴: A61K 9/16 , A61K 31/44

(30) Priority: 14.07.88 JP 175835/88

(43) Date of publication of application:
17.01.90 Bulletin 90/03

(84) Designated Contracting States:
BE CH DE ES FR GB IT LI NL SE

(71) Applicant: KYORIN SEIYAKU KABUSHIKI
KAISHA
2-5, Kanda Surugadai
Chiyoda-ku Tokyo(JP)

(72) Inventor: Masakatsu, Komuro
5905-58, Oaza Tomonuma Nogi-machi
Shimotsuga-gun Tochigi-ken(JP)
Inventor: Ohashi, Mitsuo
1674-2, Suna
Omiya-shi Saitama-ken(JP)

(74) Representative: Vossius & Partner
Siebertstrasse 4 P.O. Box 86 07 67
D-8000 München 86(DE)

(54) Ibudilast lipid microsphere and method of preparation thereof.

(57) Novel lipid microspheres are described which contain ibudilast as an active ingredient. They are useful as a remedy for cerebrovascular disorders and bronchial asthma. Furthermore, processes for the preparation of said lipid microshperes are described.

EP 0 350 913 A1

# IBUDILAST LIPID MICROSHPERE AND METHOD OF PREPARATION THEREOF

The present invention relates to a lipid microsphere (very small particle lipid emulsion) containing ibudilast and methods for preparing the same. More particularly, the invention relates to a novel lipid microsphere containing ibudilast as an active ingredient which is useful as a remedy for cerebrovascular disorders and bronchial asthma, and processes for preparing the lipid microsphere.

Ibudilast (3-isobutyryl-2-isopropylpyrazolo [1,5-a]pyridine is known as a compound first discovered by Irikura et al. (Japanese Patent Publication No. Sho 59-29318 corresponding to U.S. Patent 3,850,941, U.K. Patent 1,378,375 and so on). The compound is useful for prevention and treatment of bronchial asthma. This compound is also known as an antiallergic agent for, e.g., eye diseases and as an antirheumatic agent. Usefulness as a remedy for cerebrovascular disorder is also reported.

The known preparations containing ibudilast, are oral or rectal controlled release dosage forms (Japanese laid-Open Patent Application No. Sho 60-193913 corresponding to EP-A-0 156 243). Further, inhalants and transdermal absorptive drug formulations have been investigated.

However, ibudilast is so scarcely soluble in water (solubility in water is not more than 0.015 w/v%), and therefore the application of ibudilast as liquid dosage forms has been restricted. In addition the peculiar bitter taste of ibudilast causes problems when it is used in a spray formulation applied into the oral cavity or in a drop formulation applied to the nasal cavity.

Thus, the technical problem underlying the present invention is to provide a highly concentrated liquid preparation of ibudilast. The solution to this technical problem is achieved by providing ibudilast in the form of lipid microspheres. Thus, the present invention relates to novel lipid microspheres of a known compound.

Various conventional methods may be employed in preparing the lipid microsphere of the present invention. For example, oils such as soybean oil, cotton seed oil or dl-α-tocopherol (vitamin E), an emulsifier such as soybean lecithin or egg yolk lecithin, and an isotonicity adjusting agent such as glycerin are mixed and emulsified together with water. For emulsifying said mixture, usual methods may be employed such as using a homogenizing mixer, a) Manton-Gaulin homogenizer or a ultrasonic homogenizer. The mean particle size of the obtained lipid microspheres is not more than 1μ. They exhibit high long-term stability.

For the preparation of the lipid microsphere of ibudilast according to the present invention, 0.1 - 10 wt% ibudilast, 1.0 - 20 wt% oil, 0.1 - 5 wt% emulsifier, and an appropriate amount of water are mixed and emulsified. These numerical ranges are selected for the medical effectiveness and stability of the lipid microsphere.

The lipid microspheres of the present invention can be parentally administered and their use may be extended to drugs for intravenous injection, skin absorptives and eye drops. In addition, the bitter taste of ibudilast is significantly reduced in the microspheres of the present invention. This permits the application of those ibudilast lipid microspheres into the oral cavity or the nasal cavity by spraying or dropping.

Detailed Description of the Preferred Embodiments:

The lipid microspheres of the present invention containing ibudilast and methods for their preparation are demonstrated in detail in the following illustrating examples:

Example 1.

1 .0g of ibudilast was added to a mixture consisting of 10 g of soybean oil, 1.2 g of soybean lecithin and 2.5 g of glycerin.

The solution was thoroughly mixed and purified water was added to a total volume of 100 ml. This mixture was then emulsified with a homogenizing mixer in order to produce lipid microspheres which contain 1 w/v% of ibudilast.

Example 2

1.0 g of ibudilast was added to a mixture consisting of 10 g of dl-α-tocopherol (vitamin E), 1.2g of soybean lecithin and 2.5 g of glycerin. The solution was thoroughly mixed and purified water was added to a total volume of 200 ml. The resulting mixture was emulsified with a homogenizing mixer in order to produce lipid microspheres which contain 0.5 w/v% of ibudilast.

Example 3

1.0 g of ibudilast was added to a mixture consisting of 10 g of cotton seed oil, 1.2 g of soybean lecithin and 2.5 g of glycerin. The solution was thoroughly mixed and purified water was added to a total volume of 100 ml. This mixture was emulsified with a homogenizing mixer in order to produce lipid microspheres containing 1 w/v% of ibudilast.

ing agent is glycerin.

Example 4

1.0 g of ibudilast was added to a mixture consisting of 10 g of soybean oil, 1.2 g of egg yolk lecithin and 2.5 g of glycerin. The solution was thoroughly mixed and purified water was added to a total volume of 100 ml. The mixture was emulsified with a homogenizing mixer in order to produce lipid microspheres containing 1 w/v% of ibudilast.

Example 5

5.0 g of ibudilast were added to a mixture consisting of 10 g of soybean oil, 1.2 g of egg yolk lecithin and 2.5 g of glycerin. The solution was thoroughly mixed and purified water was added to a total volume of 100 ml. The mixture was emulsified with a homogenizing mixer in order to produce lipid microspheres containing 5 w/v% of ibudilast.

The ibudilast lipid microspheres of the present invention have excellent properties such as high distribution in tissues of the human body after systemic (oral or intravenous) administration and a lowered incidence of side effects. Therefore, said lipid microspheres can be advantageously used various dosage forms.

**Claims**

1. A lipid microsphere containing ibudilast (3-isobutyryl-2-isopropylpyrazolo[1,5-a]pyridine).

2. Method for preparing a lipid microsphere containing ibudilast (3-isobutyryl-2-isopropylpyrazolo [1,5-a]pyridine), which comprises mixing 0.1 to 10 wt% of ibudilast, 1.0 to 20 wt% of an oil, 0.1 to 5 wt% of an emulsifier and an adequate amount of water to emulsify the mixture.

3. Method for preparing a lipid microsphere according to claim 2, wherein an isotonicity adjusting agent is added to the mixture.

4. Method for preparing a lipid microsphere according to claim 2 or 3, wherein the oil is soybean oil, cotton seed oil or dl-α-tocopherol (vitamin E).

5. Method for preparing a lipid microsphere according to claim 2, 3 or 4, wherein the emulsifier is soybean lecithin or egg yolk lecithin or a mixture thereof.

6. Method for preparing a lipid microsphere according to claim 3, wherein the isotonicity adjust-

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 104, no. 17, 28th April 1986, page 41, abstract nr. 141928m, Columbus, Ohio, US; S. HARA et al.: "Antigenicity studies of 3-isobutyryl-2-isopro-pylpyrazolo[1,5-a] pyridine (KC-404) in guinea pigs, rabbits and mice", & OYO YAKURI 1985, 30(6), 1045-56 --- | 1 | A 61 K 9/16 A 61 K 31/44 |
| A | EP-A-0 118 916 (KYORIN) * Whole document * ----- | 1 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 20-10-1989 | SCARPONI U. |